# EUROPEAN PATENT APPLICATION

(11) **EP 1 314 786 A1**
(43) Date of publication of application: **28.05.2003**
(21) Application number: 00955060.9
(22) Date of filing: 28.08.2000
(51) Int. Cl.: C12Q 1/32, G01N 27/30, G01N 33/68

(54) **METHOD OF EXAMINING DISEASES WITH INBORN ERRORS OF METABOLISM AND EXAMINATION APPARATUS THEREFOR**

(71) Applicant: Sapporo Immuno Diagnostic Laboratory, Sapporo-shi, Hokkaido 001-0922 (JP)
(72) Inventor: YOKOYAMA, Toru, Sapporo Immuno Diagnostic Lab., Sapporo-shi, Hokkaido 001-0922 (JP); SHINOZUKA, Naoki, Sapporo Immuno Diagnostic Lab., Sapporo-shi, Hokkaido 001-0922 (JP); NAKAMURA, Kenji, Sapporo Immuno Diagnostic Lab., Sapporo-shi, Hokkaido 001-0922 (JP)
(74) Representative: HOFFMANN - EITLE
(86) International application number: JP0005788
(87) International publication number: WO02018627

(57) **Abstract**

The present invention achieves convenient, rapid tests for three diseases, galactosemia, maple syrup urine disease, and phenylketonuria, which are inborn errors of metabolism. This invention is a testing method and a testing apparatus which use, at least as reaction reagents, respective dehydrogenases specific for D-galactose, branched chain amino acids including L-leucine, and L-phenylalanine, as assay target substances in a biological sample, a coenzyme, an electron mediator, and a tetrazolium salt, and which detect formazan, dependent on the concentration of the target substance and finally formed by an enzyme reaction and an oxidation-reduction reaction between the biological sample and the reaction reagents, by means of an optical detection method and/or an electrochemical detection method, thereby determining the target substances conveniently and rapidly.

## Description

### FIELD OF THE INVENTION

This invention relates to a measuring method and a measuring apparatus capable of simple and prompt determination of D-galactose, branched chain amino acids including L-leucine, and L-phenylalanine, which are contained in a biological sample, without requiring tiresome pretreatment.

Particularly in three diseases, galactosemia, maple syrup urine disease, and phenylketonuria, which are inborn errors of metabolism, the method and the apparatus are a testing method and a testing apparatus which can be used in neonatal screening for early detection of these diseases, bedside examination of patients with these diseases, and monitoring in their daily lives.

### BACKGROUND OF THE INVENTION

Worldwide spread of neonatal screening intended for early detection of inborn errors of metabolism began with the discovery of therapies for phenylketonuria (PKU) and semiquantitative determination of L-phenylalanine (L-Phe) in dried blood spot on a filter paper.

A major reason why inborn errors of metabolism became target diseases for screening is the elucidation of medical advantages, such that early detection can be expected to result in normal development of children with the diseases, and high economical efficiency obtained thereby. On one hand, there is a case in which delayed detection produces handicapped children; accommodation centers or institutions for them are established; and huge medical expenses are paid for care of the diseased children. On the other hand, there is a case where early detection and treatment grow the impaired children into healthy adults. Comparison shows that the latter case is extremely high in economical efficiency, and improves the QOL of the diseased children.

Hence, the prevention of mental retardation and disturbed development due to inborn errors of metabolism is recognized in different countries as a challenge in the field of public health, and programs for neonatal screening are implemented on the national level and with governmental support.

PKU, representative of inborn errors of metabolism, is a disease caused by congenital absence of L-Phe hydroxylase which converts L-Phe, an essential amino acid, into tyrosine. In this disease, L-Phe is accumulated in vivo, and a large amount of phenylpyruvic acid as well as L-Phe is excreted in the urine. Clinical symptoms include intellectual disturbance such as mental deficiency, neuropathy, and amelanosis. To treat this disease, dietetic therapy restricting L-Phe intake is needed, and this treatment has to be continued at least up to adulthood, and preferably throughout life. Since L-Phe is one of essential amino acids for the human body, its intake must be strictly maintained within the range between the maximum amount causing no encephalopathy and the minimum amount necessary for body growth.

Maternal PKU is a state in which women with PKU, if pregnant, present fetuses with developmental disturbance, intellectual disturbance, microcephaly, or heart malformation, because they have high blood L-Phe levels. However, these anomalies can be prevented by controlling the blood L-Phe levels since before pregnancy.

Screening test methods for diagnosis include bacterial inhibition assay (BIA) developed by Guthrie et al. (Pediatrics, Vol. 32, p. 338(1963)). BIA uses Bacillus subtilis and a metabolic antagonist on a sample of blood which has been taken from a newborn's heel by puncture and allowed to seep into a filter paper. Screening was begun with the use of BIA. Further, Guthrie also found that L-histidine, L-methionine, and L-leucine (L-Leu) could be determined using the principle of BIA, and that D-galactose (D-Gal) could be assayed using Escherichia coli. Thus, screenings for histidinemia, homocystinuria, maple syrup urine disease (MSUD), and galactosemia (GE) were also started.

Basis for the current multiple screening system was established in the 1960's by the BIA method or the Paigen method (Journal of Lab. Clin. Med., Vol. 99, p. 895(1982)) which employed Escherichia coli and phage. These testing methods are very simple methods which use dried blood spot on a filter paper as a sample, and in which blood-impregnated filter paper disks punched out by a puncher are arranged on an agar culture medium, and incubated overnight, whereafter the size of bacterial growth circles is evaluated. These methods do not require expensive instruments, are excellent in terms of reagent cost, and are capable of testing numerous samples for multiple items.

However, the final results of testing are obtained by evaluation based on visual inspection, thus making it difficult to ensure the objectivity, and preserve the records of, the results of evaluation. An example of an improvement on this difficulty is imaging using a camera (Journal of the Japan Society of Mass Screening, Vol. 6, p. 23(1996)), which is intended for quantification and recording of the evaluation results. However, the quantification of bacterial growth with great variations is extremely difficult, and convenience is minimal.

On the other hand, high-performance liquid chromatography (Journal of Chromatography, Vol. 274, p. 318(1983); Journal of the Japan Society of Mass Screening, Vol. 5, p. 86(1995)), and an automatic analyzer (Clinical Chemistry, Vol. 30, p. 287(1984)) are also available, but they are problematical in that the target disease is only amino acid metabolism abnormality, an expensive measuring device is used, and their convenience and rapidity are also questionable.

Recently, there has been developed a kit based on the method, called the enzyme method (Screening, Vol. 1, p. 63(1992); The Journal of Medicine and Pharmaceutical Science, Vol. 31, p. 1237(1994)) or the microplate fluorescence method (Clinical Chemistry, Vol. 35, p. 1962(1989)), in which an enzyme reaction, followed by a fluorescence reaction, is performed in a microplate, and D-Gal, branched chain amino acids including L-Leu, as well as L-Phe and L-methionine, in a sample are determined from the intensity of fluorescence (The Journal of Medicine and Pharmaceutical Science, Vol. 37, p. 1211(1997)). This kit has a high ability to treat samples, and has achieved recording and quantification, which ensures the objective evaluation of the results of testing, the advantages difficult to obtain by conventional methods. Furthermore, the kit can obtain the test results in about 3 hours, showing a marked improvement in rapidity.

As described above, methods for treating massive samples in neonatal screening are being established. However, if point-of-care testing (POCT), typified by blood glucose monitoring in patients with diabetes, is taken as an example, its convenience and rapidity leave much to be improved.

Among POCT methods, emergency examination and bedside real-time monitoring have been achieved, by simple, rapid, small instruments, as clinical tests in settings of medical care and nursing. Currently, several microliters of whole blood can be used as a sample for blood glucose determination, and the test results can be obtained in 30 seconds to 1 minute (for example, WO99/51974(1999)). The method for measurement is a method relying on either an enzyme reaction, followed by an electrochemical reaction (for example, Analytical Chemistry, Vol. 56, p. 667(1984)), or an enzyme reaction, followed by a color reaction (for example, The Journal of Medicine and Pharmaceutical Science, Vol. 39, p. 357(1998)). Either method involves a simple procedure in which a test paper containing reagents is mounted on a small measuring instrument, and the test paper is spotted with a sample, or a sample is applied as a drop onto a test paper, and the test paper is mounted on the measuring instrument.

For inborn errors of metabolism, in particular, early detection and early treatment are the most effective methods. Of them, GE and MSUD are diseases of much urgency, and are expected to present cases which govern the prognosis of the diseased children.

Thus, it is clear that if a novel testing system, which gives test results at the spot of blood sampling, is established, it can ensure supply to a huge demand.

The establishment of self-determination methods for PKU and maternal PKU tests is similarly desired.

### SUMMARY OF THE INVENTION

To solve the above-described problems, the present invention provides a testing method and a testing apparatus capable of easily and rapidly presenting test results on D-Gal, branched chain amino acids including L-Leu, and L-Phe which are assay target substances in biological samples in the three inborn errors of metabolism, GE, MSUD and PKU, respectively.

The biological samples are not limited to whole blood, serum, plasma and blood, and wide varieties of body fluids, such as urine and saliva, can be used as the samples. Not only biological samples, but also branched chain amino acids, such as D-Gal and L-Leu, and L-Phe in foods can be measured.

The principle of determination according to the present invention is to employ an enzyme reaction by a dehydrogenase, which is specific for each of D-Gal, branched chain amino acids including L-Leu, and L-Phe as substrates, and a coenzyme, and a subsequent oxidation-reduction reaction between an electron mediator and a tetrazolium salt, and detect formazan, which is a final product dependent on the concentration of the substrate and chemically stable. This method of detection makes use of the characteristics of this method, namely, a color reaction occurring in the formation of formazan, and an oxidation-reduction reaction due to the donation and acceptance of an electron during the formazan formation, and takes advantage of an optical technique and/or an electrochemical technique.

Moreover, the testing method of the present invention involves immobilizing, beforehand, reaction reagents necessary for determination, thereby obviating the need for preparation of reagents at the time of their use, and maximally simplifying a test performer's operation.

Furthermore, the testing system of the present invention comprises test papers (sensor chips) using the above testing method and having reaction reagents immobilized therein, and a compact measuring device (portable meter) having an optical detection circuit and an electrochemical detection circuit. The test papers are mounted in the measuring instrument, and a sample, which is a biological sample, is applied as a drop onto each of the test papers. Merely by this procedure, assay is completed in several minutes, and the results of tests are displayed.

In this manner, the testing system is realized which enables tests to be conducted at the spot of taking a biological sample and which gives test results rapidly by simple procedure.

### DESCRIPTION OF THE INVENTION

According to the present invention, there are provided a testing method and a testing apparatus which use, as substrates, D-Gal, branched chain amino acids including L-Leu, and L-Phe being assay target substances in a biological sample in the three inborn errors of metabolism, GE, MSUD and PKU, respectively, which use, at least, dehydrogenases specific for the target substances, a coenzyme, an electron mediator and a tetrazolium salt as reaction reagents, and which calculate formazan, a final product dependent on the concentration of the substrate, as the concentration of the substrate by utilizing optical detection and/or electrochemical detection.

The convenient and rapid testing method for the three diseases in the present invention is characterized by immobilizing the reaction reagents beforehand onto the site of reactions, for example, a cuvette, a tube, a well or a cassette, and applying a biological specimen, a sample, as a drop onto the site of reactions, thereby dissolving and mixing the immobilized reagents to proceed with an enzyme reaction and an oxidation-reduction reaction sequentially. Then, formazan, a final product, is determined by use of optical detection and/or electrochemical detection.

The testing apparatus for the three diseases in the present invention comprises reaction reagent-immobilized test papers (sensor chips) which have been produced by using the above-described testing method, and a portable, compact measuring device (portable meter) having an optical detection circuit and an electrochemical detection circuit.

The reaction reagent-immobilized test paper is characterized by a structure which has rapid absorbency and enhances solubility of the immobilized reaction reagents when the sample is applied as a drop. The test paper is designed such that the sample is spread from above toward below, and the test paper has a layered structure. In the test paper, an absorption layer, a spread layer, and a reaction layer are provided, and materials suitable for the respective uses are selected. Because of this layered structure, the resulting test paper is satisfactory in the ability to separate the sample. As for whole blood, colored components which seem to be blood cells do not appear in the lowest layer.

The optical detection circuit in the compact measuring device makes determination with the absorbance of formazan as an indicator (Japanese Unexamined Patent Publication No. 1997-286784(1997); Analyst, Vol. 120, p. 113(1995)). The configuration of the device has been newly worked out in the present invention. Concretely, the device is composed of a light source for emitting the specific absorption wavelength of formazan, and an element for receiving reflected light after formazan exposure to incident light from the light source, and the device measures the quantity of reflected light inversely proportional to the concentration of formazan. A light emitting diode or a laser diode is used as the light source, while a photodiode or a phototransistor is used as the photo detector, whereby the optical detection circuit can be constructed from relative inexpensive semiconductor devices.

Next, the electrochemical detection circuit applies an oxidation potential specific for formazan to an electrode system, and measures an electrolytic oxidation current occurring on this occasion. Its structure is composed of a circuit for applying a constant potential, and a circuit for measuring a response current. This detection circuit can also be downsized at a low cost with the use of semiconductor devices.

By possessing the two types of detection methods, the optical one and the electrochemical one, the advantages of both methods can be utilized. If large amounts of oxidation-reduction substances, i.e., contaminants, are contained in the sample, an electrochemical background response is enhanced, and the response which should be found is impeded. In such a case, the optical detection method is suitable. In the case of a deep color or marked turbidity, on the other hand, the electrochemical method of detection is advantageous. The detection in the present invention is greatly characterized in that the above two methods are adopted to compensate for drawbacks in the assay, thereby remarkably improving the reliability of the assay and the test results.

The electrode system forming the basic structure of the reaction reagent-immobilized test paper uses the principle of reaction and manufacturing technologies already invented by us (Japanese Unexamined Patent Publication No. 2000-35413(2000), WO00/04378(2000), PCT/JP99/01392(1999)). The electrode system comprises, at least, a working electrode and a counter electrode which are formed with the use of conductive materials. The layered structure having the reaction reagents immobilized therein is disposed on the upper surface of the electrode reaction portion.

The advantages obtained by use of the testing method and the testing apparatus for inborn errors of metabolism in the present invention will be described as follows: Since the reaction reagents are immobilized beforehand, it is not necessary for the performer of tests to prepare reagents. No pretreatment is needed. Since the reaction reagent-immobilized test papers and the measuring device are utilized, extensive equipment is not required. Anyone can carry out the procedure without need to be skilled in techniques, because simply by mounting the test papers on the measuring device and depositing a sample on the test papers, measurements automatically start, and the results are automatically displayed.

The concrete use of the testing method and apparatus is in a testing system for neonatal screening which has spread worldwide. Usually, the testing system requires a period of about 1 week for blood sampling, mailing of the sample, such as a filter paper with sampled blood, testing at a screening center, and reporting of the test results. The testing method and apparatus of the present invention eliminate this required period, and permit tests to be conducted at the spot of sampling. In consideration of labor costs, spending on new plant and equipment, and maintenance and management, immeasurable economic effects can be expected. In some countries, where no screening system for newborns has not been established, testing can be started at once by introducing the present testing system which requires no special facilities or equipment.

Compared with the current testing instruments in neonatal screening, the present testing system is very compact and inexpensive, so that it can be deployed at departments of obstetrics, gynecology and pediatrics, and can further be put to private home use as well as portable use.

For GE and MSUD, which are diseases requiring urgent action, test results can be promptly presented, whereby immediate, appropriate treatment can be performed to lead the prognosis of the diseased children to optimal outcome.

As described above, the combination of one measuring device (portable meter) and three types of dedicated test papers (sensor chips) provides the present testing system as a tool for neonatal screening which can be spread worldwide, and as a tool for convenient, rapid testing in sick children and patients with inborn errors of metabolism.

### BRIEF DESCRIPTION OF THE DRAWINGS

FIG. 1 is a reaction scheme according to the present invention;

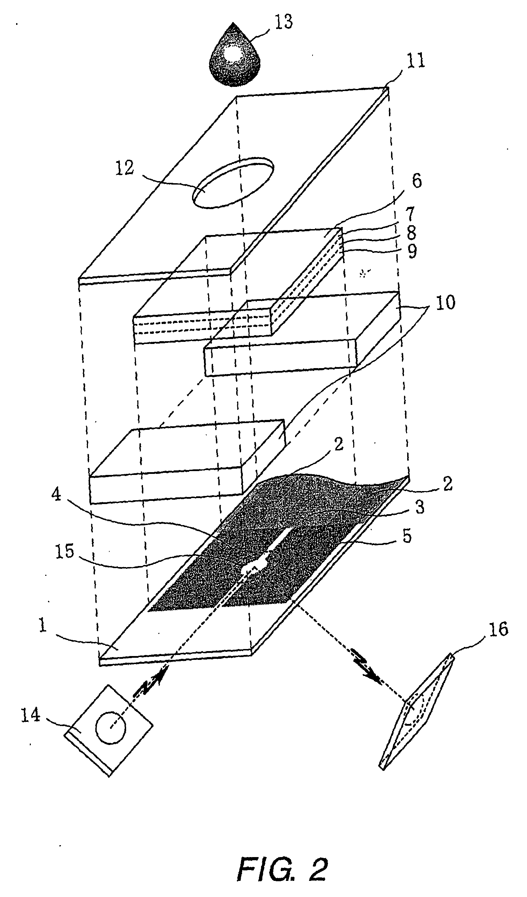

FIG. 2 is an exploded perspective view of main constituents of a sensor chip in an Example;

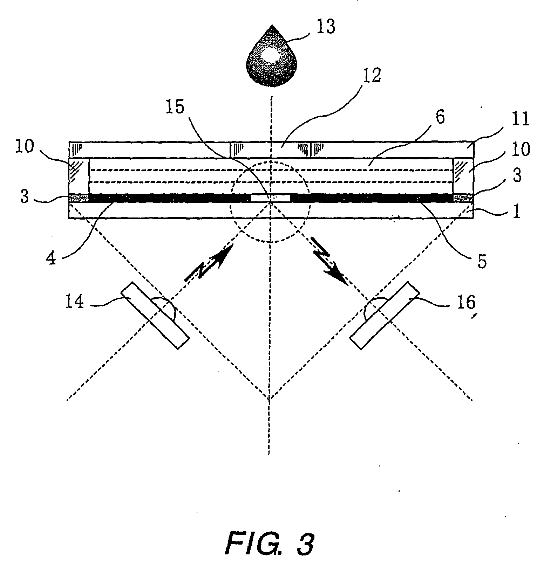

FIG. 3 is a sectional view of the sensor chip in FIG. 2; and
FIG. 4 is a constitution drawing of the sensor chip, a portable meter, and internal circuits of the portable meter.

The numerals in the drawings are defined as follows:
1, an insulating (transparent) board; 2, a lead; 3, an insulating layer; 4, a working electrode; 5, a counter electrode; 6, a reaction reagent-immobilized layer; 7, an absorption layer; 8, a spread layer; 9, a reaction layer; 10, a spacer; 11, a cover; 12, a sample supply port; 13, a sample; 14, a light source; 15, an optical detection window; 16, a photo detector; 17, a sensor chip; 18, a portable meter; 19, a chip insertion port; 20, a liquid crystal display screen; 21, a switch; 22, a microcomputer; 23, the light source 14 and a potential application circuit; 24, the photo detector 16 and an response current measurement circuit; 25, a timer; 26, a buzzer; and 27, a battery.

### DESCRIPTION OF THE PREFERRED EXAMPLES

In the present invention, the contents concerning the testing method, and the contents concerned with the testing apparatus will be described in this order. First, the reaction reagents in the testing method are described below.

The dehydrogenases used in the present invention are not restricted, if they are enzymes which use D-Gal, branched chain amino acids including L-Leu, and L-Phe as substrates and which further form reduced coenzymes. Nor are their origins restricted.

The electron mediator is not restricted, as long as it is a substance which promptly performs an oxidation-reduction reaction with the reduced coenzyme and a tetrazolium salt. For example, quinones, diaphorase, cytochromes, viologens, phenazines, phenoxazines, phenothiazines, ferricyanides, ferredoxins, ferrocene and derivatives thereof can be used. Of these compounds, phenazines show stability of response. 1-Methoxy PMS, in particular, has high storage stability and excellent reactivity with the reduced coenzyme and the tetrazolium salt, and thus has been found to be preferred as the electron mediator in the present invention.

The tetrazolium salt is not restricted, as long as it forms formazan. Among others, 2-(4-iodophenyl)-3 -(4-nitrophenyl)-5-(2,4-disulfophenyl)-2H-tetrazolium monosodium salt (WST-1) has been found to be preferred as the tetrazolium salt in the present invention. This is because formazan formed when WST-1 is reduced is water-soluble and chemically stable, and the resulting formazan shows a satisfactory response in the optical and electrochemical detection methods of the present invention.

Since the above-described reaction reagents are immobilized beforehand, it is possible to construct a method in which the reaction reagents, merely upon contact with a biological sample, dissolve and mix to proceed with a series of reactions, thereby forming formazan eventually. This method can be realized by coating solutions of the reaction reagents onto the site of reactions and drying them. After immobilization, their storage stability is improved by protecting them from light and dehumidifying them.

Next, the contents concerned with the testing apparatus will be described below.

The test papers (sensor chips) used in the present invention, which have a layered structure having the above-mentioned reaction reagents immobilized therein, utilize formazan, finally formed by reaction with a biological sample, by an optical detection method and/or an electrochemical detection method in combination.

The electrode system used in electrochemical detection according to the present invention is not limited, if it is an electrically conductive material and if it is electrochemically stable. Examples of the material are carbon, gold, silver, silver/silver chloride, nickel, platinum, platinum black, and palladium, and their alloys. Of them, various materials were studied, and it was found that the carbon material is inexpensive and chemically stable, and thus is preferred as the working electrode of the electrode system in the present invention.

The carbon material refers to carbon-containing materials as a whole. The usable carbon material is not limited, but may be any materials used in conventional carbon electrodes. For example, carbon fiber, carbon black, carbon paste, glassy carbon, and graphite can be used.

Such a carbon material is formed as an electrode portion on an insulating board in the customary manner. Usually, a paste of the carbon material formed using a resin binder or the like is screen printed onto the board, and dried by heating, whereby the electrode portion can be formed.

The method of printing is not limited to screen printing, and other method, such as gravure printing, offset printing or ink jet printing, can be applied.

On the precondition that the electrode system is a two-electrode system in which the counter electrode serves concurrently as a reference electrode, silver/silver chloride is selected for the counter electrode. Also, silver with the highest conductivity is selected for the lead, a junction between the electrode reaction portion and the electrochemical detection circuit. The lead is formed by screen printing.

Examples of the insulating board are glass, glass fabric based epoxy resin, ceramics, and plastic. However, the insulating board is not restricted, if it is a substance which is not affected during the formation of the electrode portion by printing or at the time of addition of the sample. For example, a film of a plastic such as polyester, polyethylene, polyethylene terephthalate (PET). polystyrene or polypropylene is inexpensive. Furthermore, because of adhesion to the conductive ink and good processability, the PET film has been found to be preferred.

To colorimetrically determine the color reaction of formazan in optical detection, it is necessary to select a colorless transparent material as the insulating board, and it is necessary that there be no absorption at the measuring wavelength and a window for exposure to light from the light source be provided.

The cover, the spacer, and the material for adhering them are not restricted in terms of shape or type, if they are not affected by a biological specimen, which is a sample, they hold the reaction reagent-immobilized layer, they do not impair a series of reactions, and they can protect the reaction portion from contact from the outside.

The reaction reagent-immobilized layer is composed of the three layers, absorption layer, spread layer and reaction layer, and their materials are polypropylene, nitrocellulose and polyethersulfone, respectively. The reaction reagent-immobilized layer may be of a single-layer structure, and there are no restrictions thereon, as long as this layer shows no deterioration of the reaction reagents, it is high in the ability to separate the sample, and it has high affinity for the biological sample.

The following is a description of the compact. portable measuring device (portable meter) used for assay with the sensor chips.

The portable meter can be connected to the sensor chips, has the optical detection circuit and the electrochemical detection circuit, and functions to detect the concentration of formazan, then convert it into the concentration of each substrate contained in the biological sample, and display the test results.

The optical detection circuit requires the light source for emitting the absorption wavelength of formazan, and the photo detector for reflected light.

The electrochemical detection circuit requires a circuit for applying a constant potential, and a circuit for examining a response current.

The switch is used for a power source and for various settings. Although the switch is described in the drawings, it is not necessary if automation is adopted.

### EXAMPLES

Examples of the present invention will now be described concretely, but the invention is not limited thereby.

### Example 1 Preparation of sensor chip

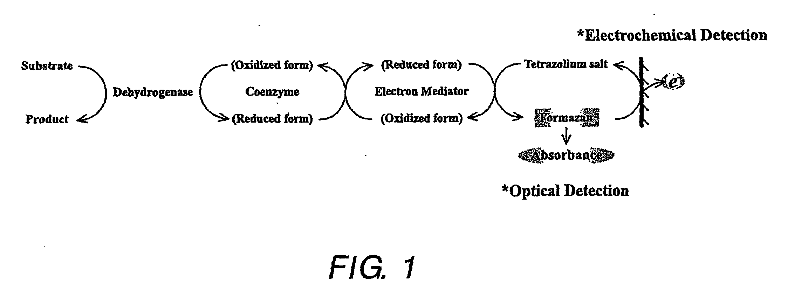

FIG. 1 shows an example of preparation of a sensor chip, and is an exploded perspective view of the main components. The sensor chip is composed of a transparent window 15 for optical detection, an electrode system formed by screen printing, a layered structure 6 having reaction reagents immobilized therein, spacers 10, and a cover 11.

In forming the electrode system, an electrically conductive silver ink (Nippon Acheson) was screen printed onto an insulating board 1 of a PET film (TORAY INDUSTRIES), and then dried by heating (120°C, 15 minutes) to form leads 2. Then, an insulating layer 3 was formed (cured by ultraviolet radiation) with the use of an insulating ink (Nippon Acheson), with portions connected to a working electrode 4 and a counter electrode 5 being left behind. Thereon, the working electrode 4 was screen printed using a conductive graphite ink (Nippon Acheson), and the counter electrode 5 was screen printed using a conductive silver/silver chloride ink (Nippon Acheson). The working electrode 4 and the counter electrode 5 were dried by heating (120°C, 15 minutes). In this manner, the electrode system was laminated by printing.

Then, in the reaction reagent immobilized layer 6, a buffer component for adjusting the optimal pH of an enzyme reaction was absorbed as a solution by a polypropylene membrane (Nippon Millipore), an absorption layer 7, and dried (40°C, 15 minutes) for immobilization.

1-Methoxy PMS (Dojin Kagaku Kenkyujo), an electron mediator, was dissolved in ultrapure water, then absorbed by a nitrocellulose membrane (Nippon Millipore), a spread layer 8, and dried (40°C, 15 minutes) for immobilization.

WST-1 (Dojin Kagaku Kenkyujo) as a tetrazolium salt, a dehydrogenase corresponding to each of substrates, and oxidized nicotinamide adenine dinucleotide (NAD⁺) (Oriental Yeast) as a coenzyme were dissolved in phosphate buffer (pH 7.4, 20 mM), then absorbed by a polyethersulfone membrane (Nippon Pall), a reaction layer 9, and dried (40°C, 15 minutes) for immobilization.

The reaction reagent immobilized layer 6, the layered structure having the required reaction reagents immobilized therein, was placed, sandwiched between spacers 10, on the electrodes formed by printing. The composite was covered with a cover 11 to produce a sensor chip 17.

### Example 2 Basic response of sensor chip

Dedicated sensor chips for GE, MSUD and PKU testing were prepared in accordance with the manufacturing procedure in Example 1. Only dehydrogenases specific for D-Gal, branched chain amino acids including L-Leu, and L-Phe as substrates were changed, and all of the other reaction reagents were the same.

For the GE testing sensor chip, D-Gal dehydrogenase (EC 1.1.1.48, Roche Diagnostics) was used.

Determination of phosphorylated Gal (galactose 1-phosphate) as well as Gal can be made by the coexistence of alkaline phosphatase (EC 3.1.3.1).

For the MSUD testing sensor chip, L-Leu dehydrogenase (EC 1.4.1.9, TOYOBO) was used. For the PKU testing sensor chip, L-Phe dehydrogenase (EC 1.4.1.20, UNITIKA) was used.

The basic responses of the three types of sensor chips were obtained using standard solutions of the respective substrates prepared in various concentrations.

Optical detection was performed by directing visible light of an arbitrary wavelength from the light source (ANDO ELECTRIC) through an optical fiber to the lowest surface of the reaction reagent immobilized layer disposed on the insulating board, and measuring the quantity of reflected light (ADVANTEST). After a sample was applied as a spot, the spotted chip was allowed to stand for 120 seconds as a time required for an enzyme reaction and an oxidation-reduction reaction. Then, the color reaction of formazan was colorimetrically measured.

In the color reaction of formazan, peak absorption wavelength is shifted depending on pH. In the pH region used in the present invention, peak absorption wavelength was confirmed in the wavelength area of about 450 nm to 600 nm.

Electrochemical detection was performed by applying the sample as a spot, then allowing the spotted chip to stand for 120 seconds as a time required for an enzyme reaction and an oxidation-reduction reaction in the same manner as described above, applying +500 mV, the oxidation potential of formazan, with the counter electrode as a reference, and measuring the oxidation current of formazan generated on this occasion (Hokuto Denko).

In the concentration region of up to 2mM. the two methods of detection and the respective sensor chips gave responses dependent on the substrate concentration.

### Example 3 Production of portable meter

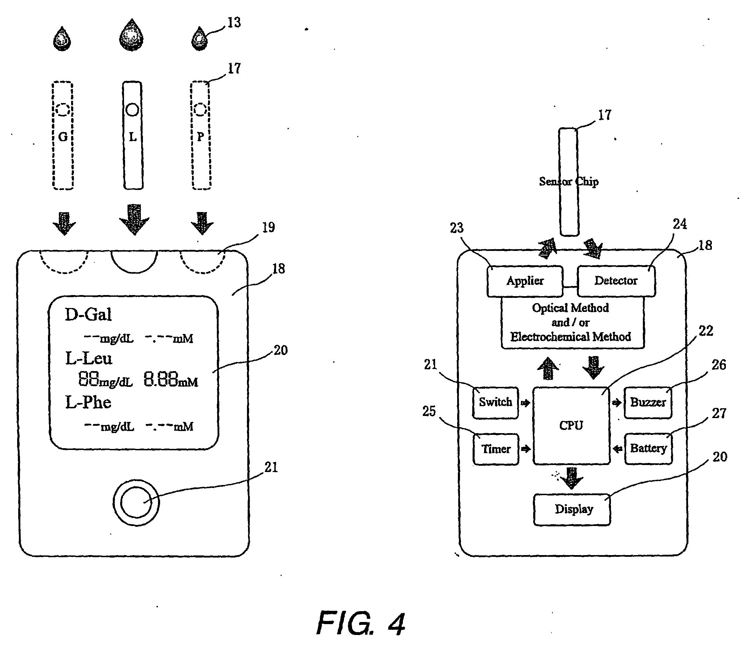

A portable meter, a measuring device using GE, MSUD and PKU testing sensor chips, is shown in FIG. 4, and assay procedure will be explained below.

Each of the sensor chips 17 is mounted on the portable meter 18 through the chip insertion port 19. At this time, actuation of the meter is not restricted; the meter may be actuated by the switch 21, or may be actuated automatically.

The sample 13 is applied as a drop onto the sensor chip 17, and measurement is started. On this occasion as well, there is no restriction as to whether start of measurement is by the switch 21 or automatic.

Using the timer 25, an enzyme reaction and an oxidation-reduction reaction are caused to proceed for a certain period of time, whereafter optical detection and electrochemical detection are performed.

The method of optical detection comprises exposing the lowest surface (optical detection window 15) of the reaction reagent immobilized layer 6 on the insulating board 1 to light from the light source for a certain period of time (corresponding to 23 of FIG. 4), and measuring the quantity of reflected light with the photo detector (corresponding to 24 of FIG. 4).

The method of electrochemical detection comprises applying a predetermined potential to the sensor chip electrode system (corresponding to 23 of FIG. 4), and measuring the value of a response current generated on this occasion (corresponding to 24 of FIG. 4).

The measured values obtained are subjected to various conversions, such as current-voltage conversion and analog-digital conversion, to calculate the substrate concentration by the microcomputer 22. The results are displayed on the liquid crystal display screen 20.

Moreover, the buzzer 26 is provided to improve operability, and the meter is driven by the battery 27 to enhance portability.

The present portable meter automatically identifies the three types of dedicated chips, executes a series of measurements, and displays the corresponding results. In testing a plurality of items, the chip insertion ports can be arranged in three channels for permitting testing of all three items nearly simultaneously for the purpose of improvements, such as simplification of tiresome sampling and reduction of a testing time by obviating the necessity for awaiting completion of measurement for each test item.

### Example 4 Basic response of sensor chips and portable meter

Basic responses were measured by use of the three types of sensor chips for GE, MSUD and PKU tests prepared in Example 1, and the portable meter produced in Example 3.

The procedure of measurement complied with Example 3, and the respective dedicated chips were mounted, whereafter standard solutions of the corresponding substrates were applied as drops. The duration of a series of reactions was 120 seconds, then optical detection was performed, and then electrochemical detection was performed consecutively. Since electrochemical detection involved electrolytic oxidation of formazan, optical detection causing no change was performed first.

Then, respective optical and electrochemical responses were compared. If the difference found satisfied certain criteria, the response was converted into the substrate concentration, and the results were displayed. However, if the difference failed to satisfy the criteria, an error was displayed.

The constitution of the optical detection circuit used a high efficiency light emitting diode (Nippon Hewlett Packard) with a peak wavelength of 590 nm as the light source, and a photodiode (Nippon Texas Instruments) as the photo detector. The electrochemical detection circuit was constituted by various semiconductor devices.

In the above examples, the particular shape was illustrated for the sensor chip, but there are no restrictions on the shape of the electrode, the arrangement of the electrodes, leads and insulating layer, the shape and disposition of the reaction reagent immobilized layer, and the shapes, arrangement and contours of the cover and the spacer.

In connection with the portable meter, the particular shape was illustrated, but this shape is not restrictive.

To achieve even higher accuracy detection, double wavelength measurement involving an additional light source for a reference wavelength in addition to the measurement wavelength is available for optical detection, and three-electrode system measurement with the addition of a reference electrode is available for electrochemical detection.

## Claims

1. A testing method capable of simultaneous tests for three diseases, galactosemia, maple syrup urine disease, and phenylketonuria, which are inborn errors of metabolism.

2. A testing method according to claim 1, permitting the simultaneous tests by simultaneously determining D-galactose, branched chain amino acids including L-leucine, and L-phenylalanine which are target substances to be measured in the galactosemia, maple syrup urine disease, and phenylketonuria.

3. A testing method according to claim 1 or 2, permitting the simultaneous tests by determining a concentration of formazan formed by an enzyme reaction and an oxidation-reduction reaction of a biological sample with reaction reagents in the determination of D-galactose, branched chain amino acids including L-leucine, and L-phenylalanine, said reaction reagents comprising, at least, each of D-galactose dehydrogenase, L-leucine dehydrogenase, and L-phenylalanine dehydrogenase, a coenzyme, an electron mediator, and a tetrazolium salt.

4. A testing method according to any one of claims 1 to 3, **characterized by** immobilizing said reaction reagents onto test papers, whereby merely upon contact with said biological sample, said reaction reagents are dissolved and mixed, and said formazan is formed via said enzyme reaction and said oxidation-reduction reaction.

5. A testing method according to any one of claims 1 to 4, **characterized in that** said formazan can be determined by using an optical method and/or an electrochemical method.

6. A testing method according to any one of claims 1 to 5, **characterized in that** said optical method measures a quantity of reflected light produced by applying an absorption wavelength of said formazan.

7. A testing method according to any one of claims 1 to 6, **characterized in that** said electrochemical method measures an response current generated by applying a certain potential to said formazan by means of an electrode system.

8. A testing apparatus having means capable of simultaneous tests for three diseases, galactosemia, maple syrup urine disease, and phenylketonuria, which are inborn errors of metabolism.

9. A testing apparatus according to claim 8, having means capable of the simultaneous tests by simultaneously determining D-galactose, branched chain amino acids including L-leucine, and L-phenylalanine which are target substances to be measured in the galactosemia, maple syrup urine disease, and phenylketonuria.

10. A testing apparatus according to claim 8 or 9, **characterized in that** said simultaneous tests for said three diseases, galactosemia, maple syrup urine disease, and phenylketonuria, use test papers and a measuring device.

11. A testing apparatus according to any one of claims 8 to 10, **characterized in that** said test papers have reaction reagents immobilized therein in said determination of D-galactose, branched chain amino acids including L-leucine, and L-phenylalanine, said reaction reagents comprising, at least, each of D-galactose dehydrogenase, L-leucine dehydrogenase, and L-phenylalanine dehydrogenase, a coenzyme, an electron mediator, and a tetrazolium salt.

12. A testing apparatus according to any one of claims 8 to 11, **characterized in that** said test papers have a window for optical detection and an electrode system for electrochemical detection.

13. A testing apparatus according to any one of claims 8 to 12, **characterized in that** said electrode system comprises, at least, a working electrode and a counter electrode.

14. A testing apparatus according to any one of claims 8 to 13, **characterized in that** said measuring device is used by having said test papers mounted thereon, and has an optical detection circuit and/or an electrochemical detection circuit.

15. A testing apparatus according to any one of claims 8 to 14, **characterized in that** said optical detection circuit comprises a light source and a photo detector.

16. A testing apparatus according to any one of claims 8 to 15, **characterized in that** said electrochemical detection circuit comprises a potential application circuit and an electric current detection circuit.

17. A testing apparatus according to any one of claims 8 to 16, **characterized in that** said measuring device has a function of automatically identifying substances mounted on a mounting portion of said device.

18. A testing apparatus according to any one of claims 8 to 17, **characterized in that** as said target substances to be measured in said determination of D-galactose, branched chain amino acids including L-leucine, and L-phenylalanine, there can be used biological samples, such as plasma, serum, whole blood, saliva and urine, food samples, and further samples which can be liquefied.
